# EUROPÄISCHE PATENTSCHRIFT

(11) **EP 2 446 922 B1**
(45) Veröffentlichungstag und Bekanntmachung des Hinweises auf die Patenterteilung: **05.03.2014**
(21) Anmeldenummer: 11184588.9
(22) Anmeldetag: 11.10.2011
(51) Int. Cl.: A61N 1/05, A61N 1/08, A61N 1/37

(54) **Implantierbare Leiter mit zusätzlichen Leitern zur Feldentkopplung**
Implantable conductors with additional, field decoupling conductors
Conducteurs implantables avec des conducteurs supplémentaires pour le découplage de champs

(30) Priorität: 28.10.2010 US 407474 P
(43) Veröffentlichungstag der Anmeldung: 02.05.2012
(73) Patentinhaber: BIOTRONIK SE & Co. KG, 12359 Berlin (DE)
(72) Erfinder: Friedrich, Michael, 14532 Kleinmachnow (DE); Maxfield, Michelle, 10967 Berlin (DE); Frenzel, Timo, 12435 Berlin (DE); Buchner, Dagmar, 10245 Berlin (DE)
(74) Vertreter: Lindner-Vogt, Karin L.

(56) Entgegenhaltungen:
- US-A1- 2008 051 854
- US-A1- 2009 099 440
- US-A1- 2009 270 956
- US-A1- 2010 256 721

## Beschreibung

Die Erfindung betrifft eine implantierbare Leitung mit einem langgestreckten Leitungskörper und einem sich in Längsrichtung des Leitungskörpers erstreckenden Funktions-Leiter, der zur Realisierung einer medizinischen Funktion der Leitung wirkt. Derartige Leitungen sind insbesondere Stimmulationselektrodenleitungen (mitunter verkürzt auch als "Elektroden" bezeichnet) von Herzschrittmachern oder Schockelektrodenleitungen von implantierbaren Defibrillatoren, es aber können auch Katheter sein, die eine langgestreckte leitfähige Struktur enthalten.

Medizinische Implantate wie die erwähnten Schrittmacher und Defibrillatoren besitzen häufig eine elektrische Verbindung zum Körperinneren des Patienten. Eine solche Verbindung dient zur Messung von elektrischen Signalen und/oder zur Stimulation von Körperzellen. Diese Verbindung ist oft als längliche Elektrodeausgeführt. Gegenwärtig werden elektrische Signale zwischen dem Implantat und den Elektrodenkontakten (Spitze, Ringe, HV-Schockwendeln, Sensoren o.Ä.) mit elektrisch gut leitenden Materialien übertragen.

Wird ein System aus Implantat und Elektrode starken Störfeldern (EMI, MRI) ausgesetzt, so kann es zu unerwünschtem Fehlverhalten kommen, speziell einer Erwärmung von Teilen des Systems oder elektrischen Fehlfunktionen (z.B. Resets). Die Erwärmung kann zu Schädigungen von Körpergewebe oder von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: die Elektrode wirkt als eine Antenne und empfängt Energie aus den umgebenden Feldern. Diese Energie auf den therapeutisch genutzten Leitungen kann die Antenne distal über die Elektrodenkontakte (Spitze, Ring ..) an das Gewebe oder proximal an das Implantat abgeben.

Die gleichen Probleme treten auch bei anderen länglichen leitfähigen Strukturen auf, deren proximales Ende nicht zwingend mit einem Implantat verbunden ist (z.B. bei Kathetern, temporäre Elektroden ).

Bekannt sind abgeschirmte Elektroden. Die Schirmung der Elektrode wirkt vor allem gegen von außen einkoppelnde elektrische Felder. Außerdem sind diese Schirmungen nur mit einer entsprechenden Schirmstärke wirksam und langzeitstabil. Es ist daher ein Kompromiss zu finden zwischen einer Vergrößerung des Elektrodendurchmessers - mit entsprechenden Auswirkungen für die Kosten und die Handlichkeit der Elektrode - und Einbußen in der Schirmwirkung.

Zur Vermeidung von Störungen durch magnetische Wechselfelder und speziell in Magnetresonanz-Apparaturen (MRI), speziell zur Begrenzung von Erwärmungen der Elektrodenspitze in solchen Feldern, wurde in der US 2008/0243218 A1 das Vorsehen eines in Längsrichtung abwechselnd hin und her geführten Schutz-Leiters in einer Elektrodenleitung vorgeschlagen. Dieses sogenannte Billabong-Prinzip nutzt ebenfalls Gegeninduktivitäten zur Minderung induzierter Ströme. Allerdings ist in diesem Fall durch die dreilagige Wendelwicklung ebenfalls eine Vergrößerung des Elektrodendurchmessers zu erwarten. Zudem wird die Elektrode eine geringere Leitfähigkeit aufweisen. Das Dokument US 2009/099440 A1 offenbart eine Leitung mit Funkstionsleitern und mit Feldentkopplungs-Leiterschleifen.

Aus Ladd M., Quick H.: Reduction of resonant RF heating in intravascular catheters using coaxial chokes, Magnetic Resonance in Medicine, 2000, sind Schutzvorkehrungen gegen durch HF-Resonanzen bewirkte Erwärmungen intravaskulärer Katheter in Form äußerer Schutzdrosseln (sog. Chokes) bekannt. Derartige Chokes liegen an der Außenhülle der Elektrode und wirken gegen Oberflächenströme. Diese Lösung reduziert keine Ströme, die in die Innenwendel einkoppeln. Außerdem ist eine Vergrößerung des Elektrodendurchmessers zu erwarten, mit den o. g. Folgen.

Der Erfindung liegt die Aufgabe zugrunde, eine verbesserte implantierbare Leitung der genannten Art bereit zu stellen, die in starken äußeren Wechselmagnetfeldern verbesserte Eigenschaften aufweist und konstruktiv leicht und somit kostengünstig realisierbar ist.

Diese Aufgabe wird durch eine implantierbare Leitung mit den Merkmalen des Anspruchs 1 gelöst. Zweckmäßige Fortbildungen des Erfindungsgedankens sind Gegenstand des abhängigen Anspruchs.

Ein wesentlicher Gedanke besteht darin, den Einfluss starker äußerer Felder durch das Vorsehen einer separaten Leiterschleife in der implantierbaren Leitung zu reduzieren. Die zusätzliche Leiterschleife, die insbesondere als Gegeninduktivität wirkt, verändert die Wechselwirkung zwischen dem äußeren Feld und der Leitung so, dass sich auf der Leitung eine andere Stromverteilung ausbildet. Die ungewollten Antenneneigenschaften der Leitung verändern sich durch diese Verstimmung. Dies resultiert in einer geringeren Erwärmung distaler Leitungskontakte. Dieser Vorteil gilt für verschiedene geometrische Formen und verschiedene Lagen der Leitung.

Gemäß der Erfindung ist die vorgeschlagene Leitung ausgebildet als Elektrodenleitung mit einem ersten und zweiten Elektrodenanschluss an einem proximalen Ende und einer ersten und zweiten Reiz-, Abfühl- und/oder Schockelektrode an oder nahe einem distalen Ende und zwei sich zwischen einem Elektrodenanschluss und einer Elektrode erstreckenden gewendelten Funktionsleiten. Hierbei umfasst jede als Feldentkopplungsmittel wirkende Leiterschleife eine in den isolierten Funktions-Leiter gewickelte Feldentkopplungs-Wendel. Die Erfindung betrifft massenhaft eingesetzte Stimulations- und Schockelektrodenleitungen implantierbarer Schrittmacher oder Kardioverter und ist daher von besonderer praktischer Bedeutung. Dadurch, dass die Feldentkopplungs-Wendel in den Funktions-Leiter gewickelt wird, wird eine Vergrößerung des Leitungsdurchmessers, wie sie ansonsten bei abgeschirmten Leitungen auftritt, vermieden.

Gemäß der Erfindung ist vorgesehen, dass die Elektrodenleitung einen ersten und zweiten proximalen Elektrodenanschluss und eine ersten und zweite distale Elektrode sowie einen sich zwischen dem ersten Elektrodenanschluss und der ersten Elektrode erstreckenden ersten Funktions-Leiter und einen sich zwischen dem zweiten Elektrodenanschluss und der zweiten Elektrode erstreckenden zweiten, zum ersten Funktions-Leiter gleich- oder gegensinnig gewickelten Funktions-Leiter aufweist. Hierbei weist die als Feldentkopplungsmittel wirkende Leiterschleife mindestens eine erste, funktional unabhängige (hochohmig oder mit hoher Impedanz gegenüber dem Funktionsstrom) in den ersten Funktions-Leiter gewickelte Feldentkopplungs-Wendel und mindestens eine zweite, funktional unabhängige (hochohmig oder mit hoher Impedanz gegenüber dem Funktionsstrom) in den zweiten Funktions-Leiter gewickelte Feldentkopplungs-Wendel auf, welche proximal und distal jeweils elektrisch mit der ersten Feldentkopplungs-Wendel verbunden ist. Diese Ausgestaltung ist an weit verbreitete Produkte angepasst und daher von besonderem praktischem Nutzen.

Die Erfindung sieht vor, dass die erste Feldentkopplungs-Wendel proximal und distal elektrisch mit der zweiten Feldentkopplungs-Wendel jeweils über einen als Schleifkontakt wirkenden leitenden Ring verbunden ist, in eine zwischen dem ersten und zweiten Funktions-Leiter und somit zwischen der ersten Feldentkopplungs-Wendel und der zweiten Feldentkopplungs-Wendel liegende Isolierung eingebettet ist und im gleitendem Kontakt mit der ersten und zweiten Feldentkopplungs-Wendel steht.

In einer weiteren konstruktiven Ausgestaltung ist vorgesehen, dass am proximalen Ende der Leitung der elektrische Kontakt zwischen der ersten und zweiten Feldentkopplungs-Wendel distal von dem oder jedem Elektrodenanschluss und am distalen Ende der Leitung der elektrische Kontakt zwischen der ersten und zweiten Feldentkopplungs-Wendel proximal von dem oder jedem elektrischen Kontakt zwischen dem oder jedem Funktions-Leiter und der oder jeder Elektrode positioniert ist.

Vorteile und Zweckmäßigkeiten der Erfindung ergeben sich im übrigen aus der nachfolgenden Beschreibung von speziellen Ausführungen anhand der Figuren. Von diesen zeigen:
Fig. 1A und 1B Prinzipskizzen zur Erläuterung der Erfindung,
Fig. 2A bis 2C Darstellungen zur Erläuterung eines Ausführungsbeispiels der Erfindung und
Fig. 3 eine Detailansicht einer erfindungsgemäß ausgestalteten Elektrodenleitung.

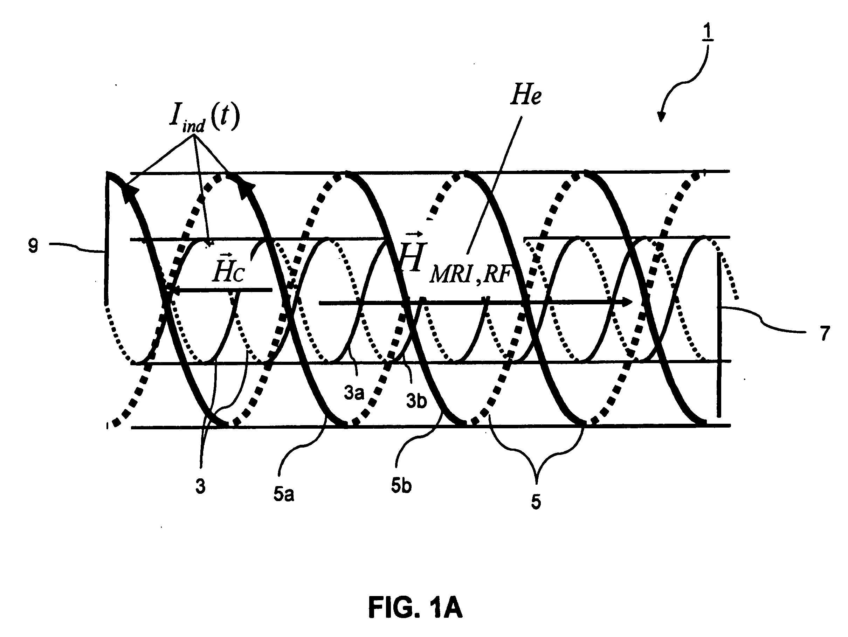

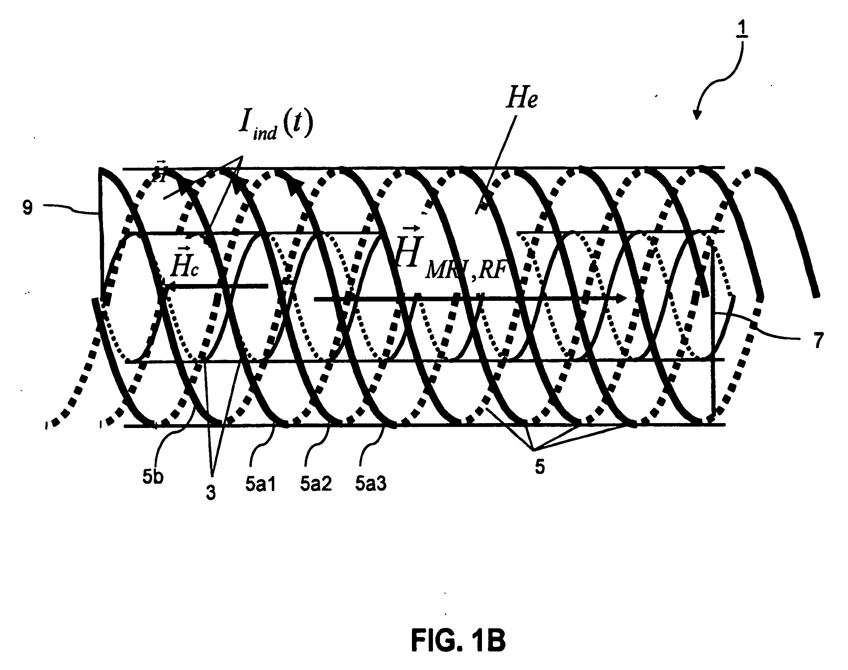

Fig. 1 ist eine Prinzipskizze zur Erläuterung der Erfindung, die nur die leitfähigen Elemente einer Elektrodenleitung 1 als Beispiel einer implantierbaren Leitung, nicht aber deren isolierenden Leitungskörper, zeigt. Bei dem dargestellten Beispiel sind ein aus zwei ineinander gewickelten Drähten gebildeter Innenleiter und ein ebenfalls aus zwei ineinander gewickelten Drähten gebildeter Außenleiter 5 vorgesehen. Innen- und Außenleiter sind bei diesem Beispiel gegensinnig gewickelt. Sie können im Gebrauch einem externen Wechselmagnetfeld Hₑ ausgesetzt sein, welches in den Leitern einen Stromfluss I(t) induziert.

Der Innenleiter (auch bezeichnet als ,,Innenwendel") 3 und der Außenleiter (auch bezeichnet als ,,Außenwendel") 5 umfassen jeweils einen Funktions-Leiter 3a bzw. 5a, der zur Realisierung einer medizinischen Funktion der Elektrodenleitung (etwa zur Übertragung von Abfühlsignalen oder Stimulations- oder Schockimpulsen) dient, und einen hiervon funktional unabhängigen Feldentkopplungs-Leiter 3b bzw. 5b, der keine medizinischen Funktion hat und insbesondere nicht der Signal- oder Impulsübertragung von bzw. an Körpergewebe eines Patienten dient. Die Feldentkopplungs-Leiter 3b, 5b sind über einen proximalen Kontakt 7 und einen distalen Kontakt 9 leitend miteinander verbunden, so dass zwischen ihnen eine geschlossene Leitungsschleife gebildet wird, in der ein Induktionsstrom fließt, welcher ein dem einwirkenden äußeren Feld Hₑ entgegenwirkendes Kompensationsfeld H_{c} erzeugt.

Fig. 1B zeigt eine modifizierte Ausführung, wobei zur Bezeichnung gleicher oder gleichwirkender Teile wie bei der ersten Ausführung die gleichen Bezugsziffern benutzt werden und eine nochmalige Erläuterung nicht erfolgt. Der wesentliche Unterschied gegenüber der ersten Ausführung besteht darin, dass der Außenleiter (die Außenwendel) 5 hier 4-fach gewendelt ist, also vier Einzeldrähte ineinander gewickelt sind, von denen die Drähte (Einzelwendeln) 5a.1, 5a.2 und 5a.3 jeweils eine medizinische Funktion haben, während der Einzeldraht 5b, wie bei der ersten Ausführung, zusammen mit dem in Gegenrichtung verlaufenden Feldentkopplungs-Leiter3b der Innenwendel 3 eine Induktionsschleife zur (partiellen) Feldentkopplung dient. Mit dieser Leitung können komplexe Abfühl-, Simulations-und Kardioversions-Funktionen bei gleichzeitigem Schutz vor übermäßiger Erwärmung der das Körpergewebe des Patienten berührenden jeweiligen Elektroden (Elektrodenkontakte) realisiert werden.

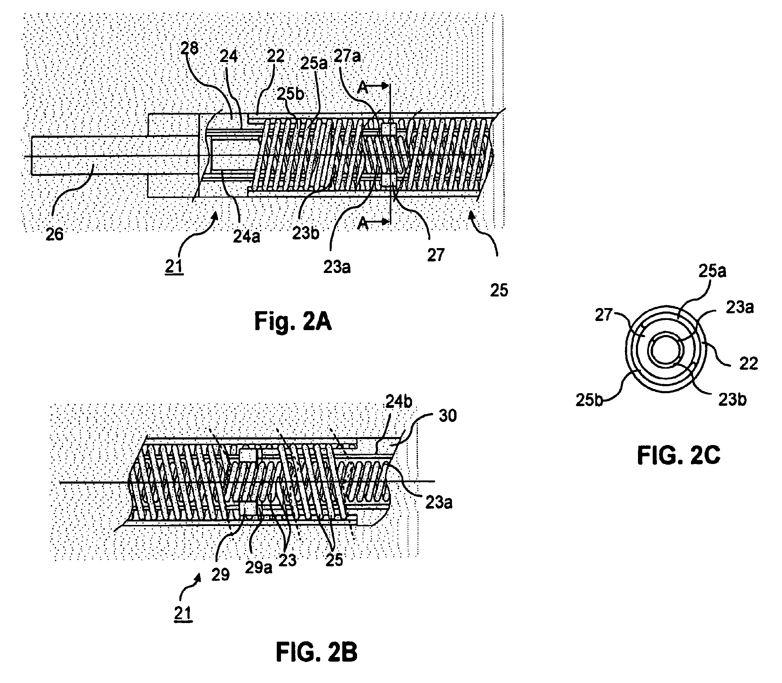

Die Figuren 2A bis 2C zeigen das Beispiel einer konstruktiven Ausgestaltung einer erfindungsgemäßen Elektrodenleitung, wobei Fig. 2A den proximalen Endabschnitt, Fig. 2B einen distalen Abschnitt und Fig. 2C einen Querschnitt zeigt und wobei der Prinzipskizze in Fig. 1A entsprechende Elemente mit an jene Figur angelehnten Bezugsziffern bezeichnet sind. Die schematische Darstellung und nachfolgenden Erläuterungen bezwecken keine vollständige Beschreibung dieser Elektrodenleitung, sondern lediglich spezieller Aspekte einer Ausführung der Erfindung.

Die Leitung 21 umfasst eine isolierende Hülle (auch bezeichnet als Leitungskörper 22, etwa aus Silikon), einen mindestens zweifach gewendelten Innenleiter 23, einen hier ebenfalls mindestens zweifach gewendelten Außenleiter 25 und eine innere und mittlere Isolierhülle 24a, 24b. Elektrodenanschlusskontakte 26, 28 dienen zum Anschluss der jeweiligen Funktions-Leiter 23a, 25a des Innenleiters 23 bzw. Außenleiters 25 an ein (nicht dargestelltes) medizinelektronisches Gerät.

Während die Funktions-Leiter 23a, 25a über ihren Längsverlauf isoliert sind, werden als Feldentkopplungs-Leiter 23b, 25b hier unisolierte ("blanke") Drähte eingesetzt, und diese stehen im proximalen Bereich der Elektrode, aber distal von den Elektrodenanschlusskontakten 26, 28, über eine Metallhülse 27 in Art eines Schleifkontaktes elektrisch miteinander in Kontakt. Der Schleifkontakt ermöglicht es, auch bei Verdrehungen und Verbiegungen der Elektrodenleitung den elektrischen Kontakt zuverlässig aufrecht zu erhalten (und damit die Induktionsschleife geschlossen zu halten), ohne dass auf die Feldentkopplungs-Leiter eine nennenswerte mechanische Belastung - wie sie unvermeidlich durch feste Kontaktstellen entstehen würden - einwirkt. Bei der dargestellten Ausführung dient eine kleine isolierende Distanzhülse 27a der elektrischen Isolierung zwischen der Metallhülse 27 und einem sich proximal hiervon erstreckenden Elektrodenanschlussbereich des Innenleiters.

Fig. 2B zeigt, dass die elektrische Kontaktierung zwischen den Feldentkopplungs-Leitern der Innenwendel 23 und Außenwendel 25 im distalen Bereich der Elektrodenleitung 21 auf die gleiche Weise wie am proximalen Ende gelöst ist, nämlich durch eine als Schleifkontakt wirkende (zweite) Metallhülse 29. Auch hier sichert eine (distal) benachbarte Distanzhülse 29a die elektrische Isolierung gegenüber einer Ringelektrode 30 der Elektrodenleitung.

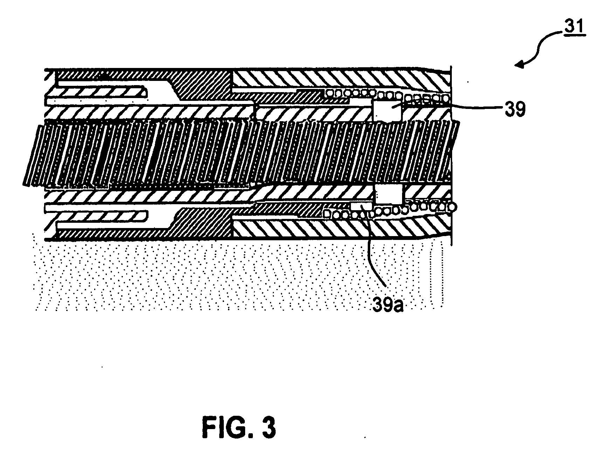

Fig. 3 zeigt nochmals das Prinzip der elektrischen Kontaktierung zwischen den Feldentkopplungs-Leitern der Innen- und Außenwendel mittels Metallhülse (Schleifkontakt) 39 mit zugeordneter isolierender Distanzhülse 39a. Die übrigen Teile der Leitung wurden hier nicht bezeichnet; diesbezüglich kann auf die obigen Erläuterungen zu Fig. 2A bis 2C verwiesen werden.

## Patentansprüche

1. Implantierbare Leitung ausgebildet als Elektrodenleitung (21) mit
- einem langgestreckten Leitungskörper (22);
- einem ersten und zweiten proximalen Elektrodenanschluss (26, 28) an einem proximalen Ende;
- einer ersten und zweiten distalen Reiz-, Abfühl- und/oder Schockelektrode an oder nahe einem distalen Ende;
- einem sich zwischen dem ersten Elektrodenanschluss und der ersten Elektrode erstreckenden ersten Funktions-Leiter (23, 23a) und einem sich zwischen dem zweiten Elektrodenanschluss und der zweiten Elektrode erstreckenden zweiten, zum ersten Funktions-Leiter gleich oder gegensinnig gewickelten Funktions-Leiter (25, 25a), der zur Realisierung einer medizinischen Funktion der Leitung wirkt; und
- als Feldentkopplungsmittel wirkenden Leiterschleifen mit einer ersten, funktional unabhängigen, insbesondere isoliert in den ersten Funktions-Leiter gewickelten, Feldentkopplunngs-Wendel (23b) und einer zweiten, funktional unabhängigen, insbesondere isoliert in den zweiten Funktions-Leiter gewickelten, Feldentkopplungs-Wendel (25b), welche proximal und distal an einer oder an mehreren Positionen über die Länge des Leitungskörpers jeweils elektrisch mit der ersten Feldentkopplungs-Wendel über einen Schleifkontakt (27, 27a, 29, 29a, 39, 39a) verbunden ist;
**dadurch gekennzeichnet, dass** der Schleifkontakt als leitenden Ring ausgebildet ist, wobei der leitende Ring in eine zwischen dem ersten und zweiten Funktions-Leiter und somit zwischen der ersten Feldentkopplungs-Wendel und der zweiten Feldentkopplungs-Wendel liegende Isolierung eingebettet ist und im gleitenden Kontakt mit der ersten und zweiten Feldentkopplungs-Wendel steht.

2. Leitung nach Anspruch 1, wobei am proximalen Ende der Leitung der elektrische Kontakt zwischen der ersten und zweiten Feldentkopplungs-Wendel (23b, 25b) distal von jedem Elektrodenanschluss (26, 28) und am distalen Ende der Leitung der elektrische Kontakt zwischen der ersten und zweiten Feldentkopplungs-Wendel proximal von jedem elektrischen Kontakt zwischen jedem Funktions-Leiter (23, 23a, 25, 25a) und der oder jeder Elektrode positioniert ist.

## Claims

1. An implantable lead designed as an electrode lead (21), comprising:
- an elongated lead body (22);
- a first and a second proximal electrode connection (26, 28) at a proximal end;
- a first and a second distal stimulation, sensing and/or shock electrode at or close to a distal end;
- a first function conductor (23, 23a) extending between the first electrode connection and the first electrode, and a second function conductor (25, 25a) extending between the second electrode connection and the second electrode and being wound in the same or opposite direction as the first function conductor, the second function conductor being provided to implement a medical function of the lead; and
- conductor loops, which act as field decoupling means, comprising a first functionally independent field decoupling coil (23b), which is wound in the first function conductor, particularly in an insulated manner, and a second functionally independent field decoupling coil (25b), which is wound in the second function conductor, particularly in an insulated manner, these field decoupling coils proximally and distally being electrically connected to the first field decoupling coil at one or more positions over the length of the lead body by way of a sliding contact (27, 27a, 29, 29a, 30, 39a),
**characterized in that**
the sliding contact is designed as a conductive ring, wherein the conductive ring is embedded in insulation located between the first and second function conductors, and hence between the first field decoupling coil and the second field decoupling coil, and is in sliding contact with the first and second field decoupling coils.

2. The lead according to claim 1, wherein at the proximal end of the lead the electrical contact is positioned between the first and second field decoupling coils (23b, 25b) distally from each electrode connection (26, 28), and at the distal end of the lead the electrical contact is positioned between the first and second field decoupling coils proximally from each electrical contact between each function conductor (23, 23a, 25a, 25a) and the or each electrode.

## Revendications

1. Conducteur implantable conçu comme un conducteur d'électrode (21) avec
- un corps conducteur allongé (22);
- des premier et deuxième raccords d'électrode proximaux (26, 28), situés à une extrémité proximale;
- des première et deuxième électrodes distales d'excitation, de détection et/ou de choc, situées à une extrémité distale ou à proximité de celle-ci;
- un premier conducteur fonctionnel (23, 23a) s'étendant entre le premier raccord d'électrode et la première électrode, et un deuxième conducteur fonctionnel (25, 25a) s'étendant entre le deuxième raccord d'électrode et la deuxième électrode, enroulé dans le même sens que le premier conducteur fonctionnel ou dans le sens inverse par rapport à celui-ci, agissant pour l'exécution d'une fonction médicale du conducteur; et
- des boucles de conducteur agissant comme des moyens de découplage de champs, avec un premier filament de découplage de champs (23b) fonctionnellement indépendant, en particulier enroulé de façon isolée dans le premier conducteur fonctionnel, ainsi qu'un deuxième filament de découplage de champs (25b) fonctionnellement indépendant, en particulier enroulé de façon isolée dans le deuxième conducteur fonctionnel, lequel est électriquement relié au premier filament de découplage de champs par un contact frotteur (27, 27a, 29, 29a, 39, 39a), de façon proximale et distale, en un ou plusieurs points sur la longueur du corps conducteur;
**caractérisé en ce que** le contact frotteur est conçu comme un anneau conducteur, l'anneau conducteur étant enrobé dans une isolation située entre le premier et le deuxième conducteurs fonctionnels et donc entre le premier filament de découplage de champs et de deuxième filament de découpage de champs, tout en étant en contact coulissant avec les premier et deuxième filaments de découplage de champs.

2. Conducteur selon la revendication 1, dans lequel, à l'extrémité proximale du conducteur, le contact électrique entre le premier et le deuxième filaments de découplage de champs (23b, 25b) est positionné de façon distale par rapport à chaque raccord d'électrode (26, 28), tandis qu'à l'extrémité distale du conducteur, le contact électrique entre le premier et le deuxième filaments de découplage de champs est positionné de façon proximale par rapport à chaque contact électrique entre chaque conducteur fonctionnel (23, 23a, 25, 25a) et l'électrode ou chaque électrode.
